# EUROPEAN PATENT SPECIFICATION

(11) **EP 4 460 363 B1**
(45) Date of publication and mention of the grant of the patent: **04.03.2026**
(21) Application number: 23704020.9
(22) Date of filing: 06.01.2023
(51) Int. Cl.: A61N 1/372, A61N 1/36

(54) **SIMPLIFED USER INTERFACE FOR MEDICAL RECHARGING DEVICE**
SIMPLIFIZIERTE BENUTZERSCHNITTSTELLE FÜR MEDIZINISCHE LADEVORRICHTUNG
INTERFACE UTILISATEUR SIMPLIFIÉE POUR UN DISPOSITIF DE RECHARGE MÉDICAL

(30) Priority: 07.01.2022 US 202263266530 P
(43) Date of publication of application: 13.11.2024
(73) Proprietor: Medtronic, Inc., Minneapolis, Minnesota 55432 (US)
(72) Inventor: FRIED, Andrew Thomas, Minneapolis, Minnesota 55432 (US); ARTHUR, Erik, Minneapolis, Minnesota 55432 (US); PARALIKAR, Kunal J., Minneapolis, Minnesota 55432 (US); SINGER, Nicholas Reid, Minneapolis, Minnesota 55432 (US); TAPANI, Sarah D., Minneapolis, Minnesota 55432 (US); HAABALA, Christopher C., Minneapolis, Minnesota 55432 (US)
(74) Representative: Zimmermann & Partner Patentanwälte mbB
(86) International application number: PCT/US2023/060208
(87) International publication number: WO 2023/133487

(56) References cited:
- US-A1- 2014 379 047
- US-A1- 2019 098 129
- US-A1- 2021 121 708

## Description

### TECHNICAL FIELD

The disclosure relates to wireless power transfer systems, and more particularly to wireless power transfer for implantable medical devices.

### BACKGROUND

Medical devices may be external or implanted and may be used to monitor patient signals such as cardiac activity, biological impedance and to deliver electrical stimulation therapy to patients via various tissue sites to treat a variety of symptoms or conditions such as chronic pain, tremor, Parkinson's disease, epilepsy, urinary or fecal incontinence, sexual dysfunction, obesity, or gastroparesis and other conditions. In some examples, medical devices may include a rechargeable electrical power source, or may be powered directly by transmitting energy through tissue. Document US 2019/098129 Al relates to an implantable device powered directly by transmitting energy through tissue and document US 2021/121708 Al relates to recharger for implantable medical devices.

### SUMMARY

The invention provides a power transmitting device according to claim 1. For better understanding of the invention the present disclosure provides also further examples of power transmitting devices, not falling under the scope of the claims. In general, the disclosure describes a user interface for a power transfer device configured to wirelessly transfer transcutaneous power to an implantable medical device. The user interface may enable a user to start and stop power transfer, e.g., to recharge a battery on the implantable medical device. In some examples, the user interface may present the user a display that indicates whether the power transfer device is performing open or closed loop recharging, indicate and control therapy delivery status of the implantable medical device and indicate both the power transfer device battery level and/or indicate the battery level for the implantable medical device. The user interface may communicate with the user with a set of indicator lights that may flash, pulse and change color as needed.

In one example, this disclosure describes a power transmitting device configured to wirelessly transfer power to a power receiving device, the power transmitting device comprising a user interface including: a control configured to receive user input: and a set of indicator lights configured to output information regarding communication status and wireless power transfer status: circuitry configured to: wirelessly communicate with the power receiving device: and wirelessly output power to the power receiving device; processing circuitry operably coupled to a memory, the processing circuitry configured to: determine one or more operational states, wherein the one or more operational states comprise at least one of: that the circuitry has not established a communication link with the power receiving device: or that the power receiving device is receiving wireless power above a threshold; selectively control based on the operational state, the set of indicator lights to perform an action, wherein the action is selected from a group consisting of: controlling at least two indicator lights of the set of indicator lights to alternately flash: controlling the set of indicator lights to pulse by increasing a brightness of one or more indicator lights of the set of indicator lights followed by decreasing the brightness of the one or more indicator lights controlling one or more indicator lights of the set of indicator lights to indicator lights to output a first color, or a second color: and controlling the set of indicator lights to output a spinning pattern.

In another example, this disclosure describes a system comprises a power receiving device; power transmitting device configured to wirelessly transfer power to a power receiving device, the power transmitting device includes a user interface including: a control configured to receive user input; and a set of indicator lights configured to output information regarding communication status and wireless power transfer status: circuitry configured to: wirelessly communicate with the power receiving device: and wirelessly output power to the power receiving device; processing circuitry operably coupled to a memory, the processing circuitry configured to: determine one or more operational states, wherein the one or more operational states comprise at least one of: that the circuitry has not established a communication link with the power receiving device, or that the power receiving device is receiving wireless power above a threshold; selectively control the set of indicator lights based on the operational state, the set of indicator lights to perform an action, wherein the action is selected from a group consisting of: controlling at least two indicator lights of the set of indicator lights to alternately flash; controlling the set of indicator lights to pulse by increasing a brightness of one or more indicator lights of the set of indicator lights followed by decreasing the brightness of the one or more indicator lights; controlling one or more indicator lights of the set of indicator lights to indicator lights to output a first color, or a second color; and controlling the set of indicator lights to output a spinning pattern.

In another example, this disclosure describes a method includes determining, by processing circuitry of a power transmitting device, one or more operational states of the power transmitting device, wherein the operational states comprise at least one of: circuitry of the power transmitting device has established a communication link with a power receiving device, wherein the power receiving device is an implantable medical device; or the power receiving device is receiving wireless power: selectively controlling, by the processing circuitry, a set of indicator lights based on the determined operational state, wherein the set of indicator lights is configured to output information regarding communication status and wireless power transfer status of the power transmitting device and wherein controlling the set of indicator lights consists of: controlling at least two indicator lights of the set of indicator lights to alternately flash; controlling the set of indicator lights to pulse by increasing a brightness of one or more indicator lights of the set of indicator lights followed by decreasing the brightness of the one or more indicator lights; controlling one or more indicator lights of the set of indicator lights to indicator lights to output a first color, or a second color; and controlling the set of indicator lights to output a spinning pattern.

The details of one or more examples of the disclosure are set forth in the accompanying drawings and the description below. Other features, objects, and advantages of the disclosure will be apparent from the description and drawings, and from the claims.

### BRIEF DESCRIPTION OF DRAWINGS

FIG. 1 is a conceptual diagram illustrating example system that includes an implantable medical device and an external charging device that charges a rechargeable power source.
FIG. 2 is a block diagram illustrating example components of the implantable medical device of FIG. 1.
FIG. 3 is a block diagram of an example external charging device of FIG 1.
FIG. 4 is a conceptual diagram illustrating an example user interface for an external charging device, according to one or more techniques of this disclosure.
FIGS. 5A and 5B are tree charts illustrating examples of indications from the user interface of the external charging device according to one or more techniques of this disclosure.
FIG. 6 is a conceptual diagram illustrating example indications from the user interface based the status of the external charging device of this disclosure.
FIG. 7 is a flow chart illustrating an example operation of an external charging device according to one or more techniques of this disclosure.

### DETAILED DESCRIPTION

The disclosure describes a user interface for a power transfer device configured to wirelessly transfer transcutaneous power to an implantable medical device. The user interface may include a control configured to receive user input, such as a power button with indicator lights that is configured to enable a user to start and stop power transfer, e.g., to recharge a battery on the implantable medical device. In some examples, the user interface may present the user a display that indicates whether the power transfer device is performing open or closed loop recharging, indicate and control therapy delivery status of the implantable medical device and indicate both the power transfer device battery level, and/or indicate the battery level for the implantable medical device. The user interface may communicate with the user via a set of indicator lights that may flash, pulse, and/or change color as needed. For example, one or more indicator lights may output a blue color when indicating the battery level of the power transfer device and a green color when indicating the battery level of the implantable medical device. In some examples, the power transfer device may communicate with an external computing device, e.g., a portable computer, a personal handset, e.g., a smart phone or a tablet computer or similar device, which may provide additional input and output functions.

In long term or chronic uses, implantable medical devices may include a rechargeable power source (e.g., one or more capacitors or batteries) that extends the operational life of the medical device to weeks, months, or even years over a nonrechargeable device. When the energy stored in the rechargeable power source has been depleted, the patient may use an external charging device to recharge the power source. In the example of an implantable medical device, the rechargeable power source is implanted in the patient and the charging device, e.g., power transfer device, is external of the patient. This charging process may be referred to as transcutaneous charging.

In some examples, transcutaneous charging may be performed via inductive coupling between a primary coil in the charging device and a secondary coil in the implantable medical device. An electrical current applied to the primary coil generates a magnetic field, and when the primary coil is aligned to the secondary coil, the magnetic field induces an electrical current in the secondary coil within the patient. A charging circuit within the implantable medical device then applies current from the secondary coil to charge the rechargeable power source within the implantable medical device. With transcutaneous transfer via inductive coils, the external charging device does not need to physically connect with the rechargeable power source for charging to occur. In some examples, the system may determine when a power transmitting and receiving system is in an inefficient position, which may cause a thermal response that less desirable than a more efficient position. The system may adjust power settings and/or provide feedback to a user to readjust to a more efficient position.

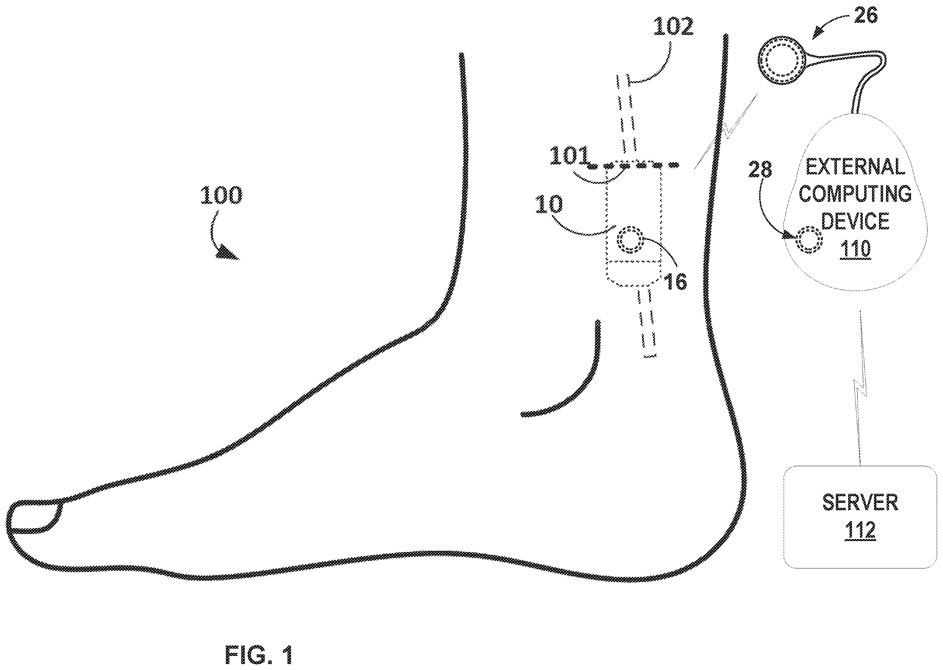

FIG. 1 is a conceptual diagram illustrating example system that includes an implantable medical device and an external charging device that charges a rechargeable power source. The example of system 100 in FIG. 1 includes an implantable medical device 10, external computing device 110, and one or more servers 112.

External computing device 110 includes one or more antenna, such as antenna 26. External computing device 110 may be used to program or adjust settings of IMD 10 and may also recharge an electrical energy storage device, such as a battery, of IMD 10. External computing device 110 may also communicate with servers 112. In other examples, an external computing device separate from external computing device 110 may communicate with IMD 10 to adjust therapy and/or sensing parameters, download recorded data, and so on.

The example of FIG. 1 is a side view of a patient's leg showing a leadless neurostimulation device 10 near the ankle adjacent to the tibial nerve 102. Device 10 can be implanted through the patient's skin and cutaneous fat layer via a small incision 101 (e.g., about one to three centimeters (cm)) above the tibial nerve on a medial aspect of the patient's ankle. While incision 101 is shown approximately horizontal to the length of the tibial nerve, other incisions or implantation techniques could be used according to physician preference. The example of FIG. 1 describes a neurostimulation implantable medical device for tibial nerve stimulation. In other examples, the techniques of this disclosure may apply to other rechargeable devices, such as implantable neurostimulation system for use in spinal cord stimulation therapy and deep brain stimulation, as well as to other types of medical devices without limitation.

Device 10 may be positioned adjacent to the region defined by flexor digitorum longus and soleus in which tibial nerve 102 is contained and implanted adjacent and proximal to a fascia layer. One or more electrodes of device 10 may face toward tibial nerve 102. Though not shown in FIG. 1, device 10 may also connect to one or more leads comprising one or more electrodes (not shown in FIG. 1).

IMD 10 may be constructed of any polymer, metal, or composite material sufficient to house the components of IMD 10. In this example, IMD 10 may be constructed with a biocompatible housing, such as titanium or stainless steel, or a polymeric material such as silicone or polyurethane, and surgically implanted at a site in patient near the tibial nerve, in some examples, while in other examples, implanted near the pelvis, abdomen, or buttocks. The housing of IMD 10 may be configured to provide a hermetic seal for components, such as a rechargeable power source. In addition, the housing of IMD 10 may be selected of a material that facilitates receiving energy to charge the rechargeable power source.

Optional testing of neurostimulation device 10 may be performed to determine if device 10 has been properly positioned in proximity to tibial nerve 102 to elicit a desired response from an applied electrical stimulation. In an example, device 10 is controlled by an external programmer to deliver test stimulation, and one or more indicative responses are monitored, such as toe flexion from simulation of the tibial motor neurons controlling the flexor hallucis brevis or flexor digitorum brevis, or a tingling sensation in the heel or sole of the foot excluding the medial arch. If such testing does not elicit appropriate motor or sensory responses, the practitioner may reposition device 10 and retest.

Once a practitioner has determined device 10 is properly positioned to provide an appropriate patient response to delivered stimulation therapy, the housing of device can be secured in place if needed. Such anchoring means may be optional as the natural shape of the region in which device 10 is implanted, and the shape of device 10 itself may have good compatibility with the surrounding tissue thus preventing device 10 from shifting or rolling after implantation. In some examples, leadless neurostimulation device 10 may further include one or more suture points to help secure device 10 to fascia or other parts of the patient. In some examples, a suture anchor may be included, such as at the distal end of the housing of device 10.

An advantage of the devices and methods described herein can be improved patient safety and therapy efficacy after implant. In contrast to other approaches, leadless neurostimulation device 10 may not require the patient's fascia layer near the implant site to be disturbed which may reduce risks affiliated with alternative procedures. Further, device 10 is a unitary structure and may be hermetically sealed.

During operation, an electrical stimulation signal may be transmitted between one or more electrodes through the fascia layer. The electrical signal may be used to stimulate tibial nerve 102 which may be useful in the treatment of overactive bladder (OAB) symptoms of urinary urgency, urinary frequency and/or urge incontinence, fecal incontinence, pain or other symptoms.

In some examples, disease, age, and injury may impair physiological functions of a patient. In one example, bladder dysfunction, such as overactive bladder, urgency, or urinary incontinence, is a problem that may afflict people of all ages, genders, and races. Various muscles, nerves, organs, and conduits within the pelvic floor cooperate to collect, store and release urine. A variety of disorders may compromise urinary tract performance, and contribute to an overactive bladder, urgency, or urinary incontinence that interferes with normal physiological function. System 100 may help relieve some symptoms of some disorders.

Urinary incontinence may include urge incontinence and stress incontinence. In some examples, urge incontinence may be caused by disorders of peripheral or central nervous systems that control bladder micturition reflexes. Some patients may also suffer from nerve disorders that prevent proper triggering and operation of the bladder, sphincter muscles or nerve disorders that lead to overactive bladder activities or urge incontinence. In some cases, urinary incontinence may be attributed to improper sphincter function, either in the internal urinary sphincter or external urinary sphincter.

One type of therapy for treating bladder dysfunction includes delivery of electrical stimulation to a target tissue site within a patient to cause a therapeutic effect during delivery of the electrical stimulation. For example, delivery of electrical stimulation from IMD 10 to a target therapy site, e.g., a tissue site that delivers stimulation to modulate activity of a tibial nerve, spinal nerve (e.g., a sacral nerve), a pudendal nerve, dorsal genital nerve, an inferior rectal nerve, a perineal nerve, or branches of any of the aforementioned nerves, may provide a therapeutic effect for bladder dysfunction, such as a desired reduction in frequency of bladder contractions. In some cases, electrical stimulation of the tibial nerve may modulate afferent nerve activities to restore urinary function.

Bladder dysfunction generally refers to a condition of improper functioning of the bladder or urinary tract, and may include, for example, an overactive bladder, urgency, or urinary incontinence. Overactive bladder (OAB) is a patient condition that may include symptoms, such as urgency, with or without urinary incontinence. Urgency is a sudden, compelling urge to urinate, and may often, though not always, be associated with urinary incontinence. Urinary incontinence refers to a condition of involuntary loss of urine, and may include urge incontinence, stress incontinence, or both stress and urge incontinence, which may be referred to as mixed urinary incontinence. As used in this disclosure, the term "urinary incontinence" includes disorders in which urination occurs when not desired, such as stress or urge incontinence. Other bladder dysfunctions may include disorders such as non-obstructive urinary retention.

In some examples, the techniques described in this disclosure are directed to delivery of neurostimulation therapy in a non-continuous manner which may include oncycles and off-cycles. For example, an IMD may deliver neurostimulation therapy for a specified period of time followed by a specified period of time when the IMD does not deliver neurostimulation (e.g., withholds delivery of neurostimulation). A period during which stimulation is delivered (an on-cycle) may include on and off periods (e.g., a duty cycle or bursts of pulses) with short inter-pulse durations of time when pulses are not delivered.

The rechargeable power source of IMD 10 may include one or more capacitors, batteries, or other components (e.g., chemical or electrical energy storage devices). Example batteries may include lithium-based batteries, nickel metal-hydride batteries, or other materials. The rechargeable power source may be replenished, refilled, or otherwise capable of increasing the amount of energy stored after energy has been depleted. The energy received from secondary coil 16 may be conditioned and/or transformed by a charging circuit. The charging circuit may then send an electrical signal used to charge the rechargeable power source when the power source is fully depleted or only partially depleted.

External computing device 110 may be used to recharge the rechargeable power source within IMD 10 implanted in the patient. External computing device 110 may be a hand-held device, a portable device, or a stationary charging system. External computing device 110 may also be referred to as charging device 110 in this disclosure. External computing device 110 may include components necessary to charge IMD 10 through tissue of the patient. External computing device 110 may include an internal energy transfer coil 28 and external energy transfer coil 26. also referred to as primary coil 26 or primary coil 28. In other examples, external computing device may only include internal primary coil 28 and omit the use of external primary coil 26, or only include external primary coil 26 and omit the use of internal primary coil 28. External computing device 110 may include a housing to enclose operational components such as a processor, memory, user interface, telemetry module, power source, and charging circuit configured to transmit energy to secondary coil 16 via energy transfer coil 26 and/or 28. Although a user may control the recharging process with a user interface of external computing device 110, external computing device 110 may alternatively be controlled by another device, e.g., an external programmer, a computing device of servers 112 such as a tablet computer, laptop or other similar computing device. The second external computing device of servers 112 may include a computing device with a touch-screen user interface. In other examples, external computing device 110 may be integrated with an external programmer, such as a patient programmer carried by the patient.

External computing device 110 and IMD 10 may utilize any wireless power transfer techniques that are capable of recharging the power source of IMD 10 when IMD 10 is implanted within the patient. In some examples, system 10 may utilize inductive coupling between primary coils (e.g., energy transfer coil 28) and secondary coils (e.g., secondary coil 16) of external computing device 110 and IMD 10. In inductive coupling, energy transfer coil 28 is placed near implanted IMD 10 such that energy transfer coil 28 is aligned with secondary coil 16 of IMD 10. External computing device 110 may then generate an electrical current in energy transfer coil 28 based on a selected power level for charging the rechargeable power source of IMD 10. When the primary and secondary coils are aligned, the electrical current in the primary coil may magnetically induce an electrical current in secondary coil 16 within IMD 10. Since the secondary coil is associated with and electrically coupled to the rechargeable power source, the induced electrical current may be used to increase the voltage, or charge level, of the rechargeable power source. Although inductive coupling is generally described herein, any type of wireless energy transfer may be used to transfer energy between external computing device 110 and IMD 10.

Energy transfer coils 26 and 28 may include a wound wire (e.g., a coil) (not shown in FIG. 1). The coil may be constructed of a wire wound in an in-plane spiral (e.g.. a disk-shaped coil). In some examples, this single or even multi-layers spiral of wire may be considered a flexible coil capable of deforming to conform with a non-planar skin surface. The coil may include wires that electrically couple the flexible coil to a power source and a charging module configured to generate an electrical current within the coil. Energy transfer coil 28 may be external of the housing of external computing device 110 such that energy transfer coil 28 can be placed on the skin of the patient proximal to IMD 10. In some examples, energy transfer coil 28 may be disposed on the outside of the housing or even within housing.

Either primary coil 26 and/or 28 of system 100 may include a heat sink device (not shown in FIG. 1). In the example of system 100, external computing device 110 is the power transmitting unit and IMD 10 is the power receiving unit. IMD 10 may be in a flipped or non-flipped position.

As noted above, in this disclosure external computing device 110 may also be referred to as recharger 110. Recharger 110 may include a user interface to receive control inputs from a user, such as the patient, medical professional or other caregiver. The user interface of recharger 110 may also provide information to a user. For example, recharger 110 may include a control configured to receive user input (not shown in FIG. 1) with as well as a set of indicator lights. In some examples the indicator lights may be configured to illuminate the control. The indicator lights may also be configured to output information regarding an operational state of external computing device 110, such as a communication status and wireless power transfer status. Processing circuitry of recharger 110 may receive an indication from the control when the control is activated, which may, for example cause the processing circuitry to turn on and turn off one or more functions of recharger 110. In some examples, the control may include a button, a switch, knobs, a touch sensitive screen or any similar component that may be manipulated by a user and signal the processing circuitry. In some examples, the indicator lights are configured to illuminate the control.

The processing circuitry determine whether IMD 10 and recharger 110 have established a communication link e.g., via communication circuitry. In response to the processing circuitry determining that recharger 110 and IMD 10 have not established a communication link, the processing circuitry may cause at least two indicator lights of the set of indicator lights to alternately flash. Recharger 110 may still wirelessly transfer power to IMD 10, but alternately flashing indicator lights may signify that recharger 110 is operating in open loop charging mode.

Processing circuitry of recharger 110 may further determine whether IMD 10 is receiving wireless power. In response to determining that IMD 10 has good power coupling, such as receiving an amount of wireless power above a power threshold, the processing circuitry may cause the set of indicator lights to pulse. In this disclosure, a "flashing" light means when an indicator light turns on and off with little or no perceptible time between the on and off state. An "occulting" light is similar to a flashing light, except that an occulting light may be ON longer than it is OFF, where for a flashing light, the light may be OFF longer than it is ON. In contrast, a pulse, or pulsing light may be described as one or more indicator lights of the set of indicator lights that increases in brightness followed by a decrease in brightness. In other words, for a pulsing light, there may be a perceptible time between the OFF and the ON state. Said another way, a pulsing light may appear to fade ON and OFF, where for flashing or occulting patterns the transition between on and off may appear to be more abrupt. In this disclosure, "perceptible time" may indicate a duration that a human may detect.

In some examples, such as for indicator lights that are light emitting diodes (LED), each indicator light may receive a time varying current, such as a pulse width modulated (PWM) current, rather than a constant current when ON and no current when OFF. The processing circuitry may cause an indicator light to receive a PWM current with frequencies ranging for example from 60 Hz to several kHz. Though the indicator light is actually flashing, the light may appear to be ON, i.e., this flashing may not perceptible because of the persistence of vision for the human eye. For a flashing or occulting pattern, the processing circuitry may cause a PWM current to start and stop as needed for the desired pattern. For a pulsing light processing circuitry may vary the duty cycle of the PWM current so indicator light appears to fade ON and OFF with a perceptible increase in brightness followed by a decrease in brightness. In some examples the changes in the duty cycle of PWM current may follow a logarithmic pattern to provide the pulsing light, because of the way the human eye perceives changes in brightness. In some examples, the set of indicator lights may include one or more RGB (Red-Green-Blue) LEDs, which may be configured to output many different colors based on the processing circuitry controlling combinations of red-blue-green color intensity.

The processing circuitry may further control the indicator lights to display how efficiently that recharger 110 is transferring power to IMD 10, e.g., a power coupling level (e.g., poor, good, excellent, and so on). In some examples, the processing circuitry may adjust the rate of pulsing of the indicator lights to indicate the coupling level. For example, the indicator lights may pulse once per second, once over a two second duration, once over a four second duration, and so on. In some examples, the pulse may have a longer duty cycle, e.g., remain lit for most of the duration. In other examples, the pulse may have a shorter duty cycle and remain lit for only a portion of the duration.

The processing circuitry may alternatively cause the indicator lights to output a series of pulses over a duration. For example, four pulses every N seconds; three pulses every N seconds, one pulse every N seconds, and so on. These are just some possible examples of indicator light output. The processing circuitry may be configured to output other types of flashing patterns, pulsing patterns or combinations of patterns.

To determine whether IMD 10 has good power coupling and is receiving wireless power, a variety of system metrics are available to external computing device 110 based on computations of power and heat done by processing circuitry of external computing device 110, as well as from metrics communicated to the recharger from IMD 10. These metrics may include but are not limited to battery current measured by IMD 10, power transfer efficiency and so on. Efficiency may be measured by IMD 10 and communicated to external computing device 110 and may be an indicator of when the recharger primary coil 28 is concentric with secondary coil 16. A concentric relative position of primary coil 28, or 26, and secondary coil 16 may be in positions with the lowest overall transient thermal response. In some examples, the energy transfer in concentric positions may be higher and the battery of IMD 10 may charge faster. In some relative positions, the time to charge may be longer so the overall thermal dose may be worse than for shorter and quicker charging periods that result from a more efficient coupling.

External computing device 110 may receive wireless communication from IMD 10 that include the amount of power delivered to the electrical energy storage device of IMD 10, which may be referred to as closed loop charging. In other words, system 10 may measure efficiency, such as IMD efficiency, to determine whether the relative position of primary coil 26 and secondary coil 16 may be in a less desirable relative position. Processing circuitry of system 100, e.g., processing circuitry of recharger 110. processing circuitry of servers 112. and/or processing circuitry of IMD 10, may calculate any of the values described herein.

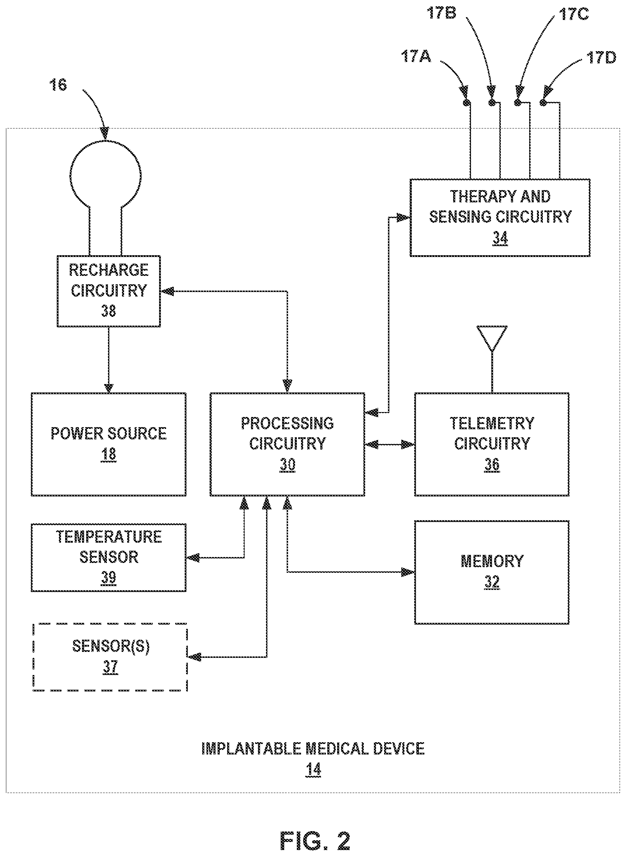

FIG. 2 is a block diagram illustrating example components of the medical device of FIG. 1. Implantable medical device 14 is an example of IMD 10 described above in relation to FIG. 1. In the example illustrated in FIG. 2, IMD 14 includes temperature sensor 39, coil 16, processing circuitry 30, therapy and sensing circuitry 34, recharge circuitry 38, memory 32, telemetry circuitry 36, power source 18, and one or more sensors 37, such as an accelerometer. In other examples. IMD 14 may include a greater or a fewer number of components. e.g., in some examples, IMD 14 may not include temperature sensor 39 or sensors 37. In general, IMD 14 may comprise any suitable arrangement of hardware, alone or in combination with software and/or firmware, to perform the various techniques described herein attributed to IMD 14 and processing circuitry 30, and any equivalents thereof.

Processing circuitry 30 of IMD 14 may include one or more processors, such as one or more microprocessors, digital signal processors (DSPs), application specific integrated circuits (ASICs), field programmable gate arrays (FPGAs), or any other equivalent integrated or discrete logic circuitry, as well as any combinations of such components. IMD 14 may include a memory 32, such as random access memory (RAM), read only memory (ROM), programmable read only memory (PROM), erasable programmable read only memory (EPROM), electronically erasable programmable read only memory (EEPROM), flash memory, comprising executable instructions for causing the processing circuitry 30 to perform the actions attributed to this circuitry. Moreover, although processing circuitry 30, therapy and sensing circuitry 34, recharge circuitry 38, telemetry circuitry 36, and temperature sensor 39 are described as separate modules, in some examples, some combination of processing circuitry 30, therapy and sensing circuitry 34, recharge circuitry 38, telemetry circuitry 36 and temperature sensor 39 are functionally integrated. In some examples, processing circuitry 30, therapy and sensing circuitry 34, recharge circuitry 38, telemetry circuitry 36, and temperature sensor 39 correspond to individual hardware units, such as ASICs, DSPs, FPGAs, or other hardware units. In this disclosure, therapy, and sensing circuitry 34 may be referred to as therapy circuitry 34, for simplicity.

Memory 32 may store therapy programs or other instructions that specify therapy parameter values for the therapy provided by therapy circuitry 34 and IMD 14. In some examples, memory 32 may also store temperature data from temperature sensor 39, instructions for recharging rechargeable power source 18, thresholds, instructions for communication between IMD 14 and an external computing device, or any other instructions required to perform tasks attributed to IMD 14. Memory 32 may be configured to store instructions for communication with and/or controlling one or more temperature sensors of temperature sensor 39. In various examples, memory 32 stores information related to determining the temperature of housing 19 and/or exterior surface(s) of housing 19 of IMD 14 based on temperatures sensed by one or more temperature sensors, such as temperature sensor 39, located within IMD 14.

For example, memory 32 may store programming settings such as electrical stimulation therapy output magnitude, pulse width, and so on. Memory 32 may determine whether a sensed bioelectrical signal is valid, such as and ECAP or other signal in response to an output electrical stimulation therapy event. Memory 32 may store programming instructions that when executed by processing circuitry 30 cause processing circuitry 30 to cause electrical stimulation circuitry therapy circuitry 34 to deliver electrical stimulation therapy to a target nerve of a patient.

Therapy and sensing circuitry 34 may generate and deliver electrical stimulation under the control of processing circuitry 30. In some examples, processing circuitry 30 controls therapy circuitry 34 by accessing memory 32 to selectively access and load at least one of the stimulation programs to therapy circuitry 34. For example, in operation, processing circuitry 30 may access memory 32 to load one of the stimulation programs to therapy circuitry 34. In such examples, relevant stimulation parameters may include a voltage amplitude, a current amplitude, a pulse rate, a pulse width, a duty cycle, or the combination of electrodes 17A, 17B. 17C, and 17D (collectively "electrodes 17") that therapy circuitry 34 may use to deliver the electrical stimulation signal as well as sense biological signals. In other examples, IMD 14 may have more or fewer electrodes than the four shown in the example of FIG. 2. In some examples electrodes 17 may be part of or attached to a housing of IMD 14, e.g.. a leadless electrode. In other examples, one or more of electrodes 17 may be part of a lead implanted in or attached to a patient to sense biological signals and/or deliver electrical stimulation, as described above in relation to FIG. 1.

In some examples, one or more electrodes connected to therapy circuitry 34 may connect to one or more sensing electrodes, e.g., attached to housing of IMD 14. In some examples the electrodes may be configured to detect the evoked motor response caused by the electrical stimulation therapy event, or other bioelectrical signals such as ECAPs, impedance and so on.

IMD 14 also includes components to receive power to recharge rechargeable power source 18 when rechargeable power source 18 has been at least partially depleted. As shown in FIG. 2, IMD 14 includes coil 16 and recharge circuitry 38 coupled to rechargeable power source 18. Recharge circuitry 38 may be configured to charge rechargeable power source 18 with the selected power level determined by either processing circuitry 30 or an external charging device, such as external computing device 110 described above in relation to FIG. 1. Recharge circuitry 38 may include any of a variety of charging and/or control circuitry configured to process or convert current induced in coil 16 into charging current to charge power source 18.

Secondary coil 16 may include a coil of wire or other device capable of inductive coupling with a primary coil disposed external to patient 12. Although secondary coil 16 is illustrated as a simple loop of in FIG. 2, secondary coil 16 may include multiple turns of conductive wire. Secondary coil 16 may include a winding of wire configured such that an electrical current can be induced within secondary coil 16 from a magnetic field. The induced electrical current may then be used to recharge rechargeable power source 18.

Recharge circuitry 38 may include one or more circuits that process, filter, convert and/or transform the electrical signal induced in the secondary coil to an electrical signal capable of recharging rechargeable power source 18. For example, in alternating current induction, recharge circuitry 38 may include a half-wave rectifier circuit and/or a full-wave rectifier circuit configured to convert alternating current from the induction to a direct current for rechargeable power source 18. The full-wave rectifier circuit may be more efficient at converting the induced energy for rechargeable power source 18. However, a half-wave rectifier circuit may be used to store energy in rechargeable power source 18 at a slower rate. In some examples, recharge circuitry 38 may include both a full-wave rectifier circuit and a half-wave rectifier circuit such that recharge circuitry 38 may switch between each circuit to control the charging rate of rechargeable power source 18 and temperature of IMD 14.

Rechargeable power source 18 may include one or more capacitors, batteries, and/or other energy storage devices. Rechargeable power source 18 may deliver operating power to the components of IMD 14. In some examples, rechargeable power source 18 may include a power generation circuit to produce the operating power. Rechargeable power source 18 may be configured to operate through many discharge and recharge cycles. Rechargeable power source 18 may also be configured to provide operational power to IMD 14 during the recharge process. In some examples, rechargeable power source 18 may be constructed with materials to reduce the amount of heat generated during charging. In other examples. IMD 14 may be constructed of materials and/or using structures that may help dissipate generated heat at rechargeable power source 18, recharge circuitry 38, and/or secondary coil 16 over a larger surface area of the housing of IMD 14.

Although rechargeable power source 18, recharge circuitry 38. and secondary coil 16 are shown as contained within the housing of IMD 14, in alternative implementations, at least one of these components may be disposed outside of the housing. For example, in some implementations, secondary coil 16 may be disposed outside of the housing of IMD 14 to facilitate better coupling between secondary coil 16 and the primary coil of external charging device. In other examples, power source 18 may be a primary power cell and IMD 14 may not include recharge circuitry 38 and recharge coil 16.

Processing circuitry 30 may also control the exchange of information with an external computing device using telemetry circuitry 36. Telemetry circuitry 36 may be configured for wireless communication using radio frequency protocols, such as BLUETOOTH, or similar RF protocols, as well as using inductive communication protocols. Telemetry circuitry 36 may include one or more antennas 37 configured to communicate with external charging device, for example. Processing circuitry 30 may transmit operational information and receive therapy programs or therapy parameter adjustments via telemetry circuitry 36. Also, in some examples, IMD 14 may communicate with other implanted devices, such as stimulators, control devices, or sensors, via telemetry circuitry 36. In addition, telemetry circuitry 36 may be configured to control the exchange of information related to sensed and/or determined temperature data, for example temperatures sensed by and/or determined from temperatures sensed using temperature sensor 39. In some examples, telemetry circuitry 36 may communicate using inductive communication, and in other examples, telemetry circuitry 36 may communicate using RF frequencies separate from the frequencies used for inductive charging.

In some examples, processing circuitry 30 may transmit additional information to external charging device related to the operation of rechargeable power source 18. For example, processing circuitry 30 may use telemetry circuitry 36 to transmit indications that rechargeable power source 18 is completely charged, rechargeable power source 18 is fully discharged, the amount of charging current output by recharge circuitry 38 e.g., to power source 18, or any other charge status of rechargeable power source 18. In some examples, processing circuitry 30 may use telemetry circuitry 36 to transmit instructions to external charging device, including instructions regarding further control of the charging session, for example instructions to lower the power level or to terminate the charging session, based on the determined temperature of the housing/external surface 19 of the IMD.

Processing circuitry 30 may also transmit information to external charging device that indicates any problems or errors with rechargeable power source 18 that may prevent rechargeable power source 18 from providing operational power to the components of IMD 14. In various examples, processing circuitry 30 may receive, through telemetry circuitry 36, instructions for algorithms, including formulas and/or values for constants to be used in the formulas, that may be used to determine the temperature of the housing 19 and/or exterior surface(s) of housing 19 of IMD 14 based on temperatures sensed by temperature sensor 39 located within IMD 14 during and after a recharging session performed on rechargeable power source 18.

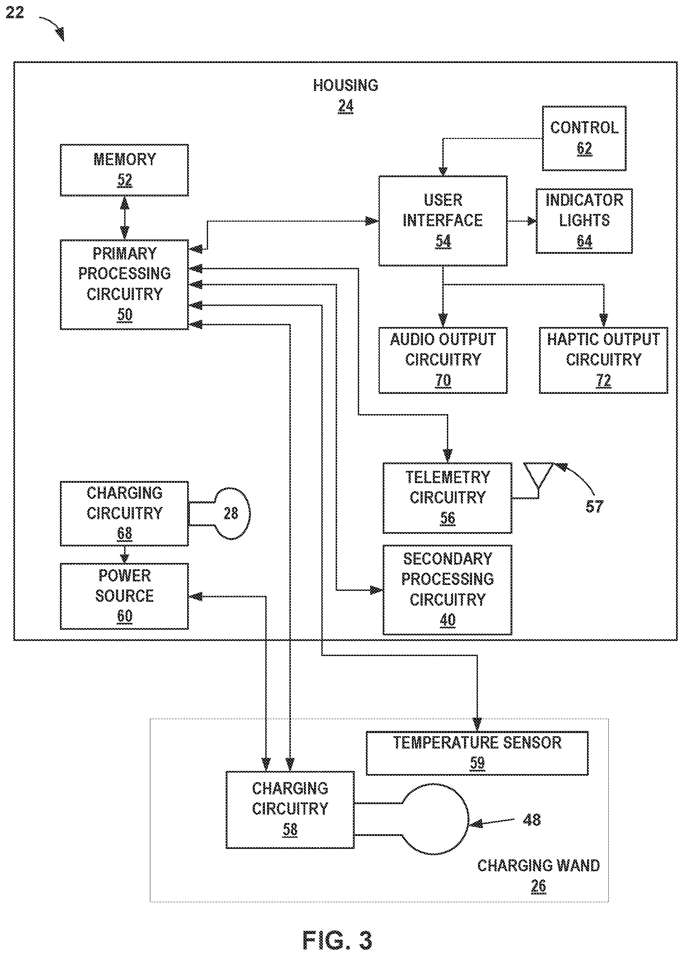

FIG. 3 is a block diagram of an example an external computing device of FIG. 1. External charging device 22 in of FIG. 2 is an example of external computing device 110 described above in relation to FIG. 1. In some examples, external charging device 22 may be described as a hand-held device. in other examples, external charging device 22 may be a larger or a non-portable device. In addition, in other examples external charging device 22 may be included as part of an external programmer or include functionality of an external programmer. As shown in the example of FIG. 3, external charging device 22 includes two separate components. Housing 24 encloses components such as a processing circuitry 50, memory 52, user interface 54, telemetry circuitry 56, control 62, one or more sets of indicator lights 64, audio output circuitry 70, haptic output circuitry 72 and power source 60. Charging head 26, also called charging wand 26, may include charging circuitry 58, temperature sensor 59, and coil 48. Housing 24 is electrically coupled to charging head 26 via charging cable 28. Housing 24 may also include charging circuitry 68 and coil 28, which is an example of coil 28 described above in relation to FIG. 1.

In some examples, separate charging wand 26 may facilitate positioning of coil 48 over coil 16 of IMD 14. In some examples, charging circuitry 68 and/or coil 28 may be integrated within housing 24 in other examples, as described above in relation to FIG. 1. In other examples, external charging device 22 may not include charging wand 26. Memory 52 may store instructions that, when executed by processing circuitry 50, causes processing circuitry 50 and external charging device 22 to provide the functionality ascribed to external charging device 22 throughout this disclosure, and/or any equivalents thereof. Coil 48 and coil 28 may also be referred to as an antenna. In some examples, external charging device 22 may include secondary processing circuitry 40, which may control telemetry circuitry 56, as well as perform other functions. Some other functions may include error checking of the operation of primary processing circuitry 50.

External charging device 22 may also include one or more temperature sensors, illustrated as temperature sensor 59, similar to temperature sensor 39 of FIG. 2. As shown in FIG. 3, temperature sensor 59 may be disposed within charging head 26. In other examples, one or more temperature sensors of temperature sensor 59 may be disposed within housing 24. For example, charging head 26 may include one or more temperature sensors positioned and configured to sense the temperature of coil 48 and/or a surface of the housing of charging head 26. In some examples, external charging device 22 may not include temperature sensor 59.

In general, external charging device 22 comprises any suitable arrangement of hardware, alone or in combination with software and/or firmware, to perform the techniques ascribed to external charging device 22. and processing circuitry 50, user interface 54, telemetry circuitry 56, and charging circuitry 58 of external charging device 22, and/or any equivalents thereof. In various examples, external charging device 22 may include one or more processors, such as one or more microprocessors, DSPs, ASICs, FPGAs, or any other equivalent integrated or discrete logic circuitry, as well as any combinations of such components. External charging device 22 also, in various examples, may include a memory 52, such as RAM, ROM, PROM, EPROM, EEPROM, flash memory, a hard disk, a CD-ROM. comprising executable instructions for causing the one or more processors to perform the actions attributed to them. Moreover, although processing circuitry 50, telemetry circuitry 56, charging circuitry 58, and temperature sensor 59 are described as separate modules, in some examples, processing circuitry 50, telemetry circuitry 56, charging circuitry 58. and/or temperature sensor 59 are functionally integrated. In some examples, processing circuitry 50. telemetry circuitry 56, charging circuitry 58, and/or temperature sensor 59 correspond to individual hardware units, such as ASICs, DSPs, FPGAs, or other hardware units.

Memory 52 may store instructions that, when executed by processing circuitry 50, cause processing circuitry 50 and external charging device 22 to provide the functionality ascribed to external charging device 22 throughout this disclosure, and/or any equivalents thereof. For example, memory 52 may include instructions that cause processing circuitry 50 to control the power level used to charge IMD 14 in response to the determined temperatures for the housing/external surface(s) of IMD 14, as communicated from IMD 14, or instructions for any other functionality. Memory 52 may include a record of selected power levels, sensed temperatures, determined temperatures, or any other data related to charging rechargeable power source 18, described above in relation to FIG. 2. Memory 52 may store instructions that when executed by processing circuitry 50 may control the operation of indicator lights 64 as described above in relation to FIG. 1. Processing circuitry 50 may determine one or more operational states, e.g., of external charging device 22 and selectively control the set of indicator lights based on the operational state. For example, memory 52 may store a table similar to table 1 below, that describes the operation of indicator lights 64 in response to communication status, therapy status and other operational states of external charging device 22 and IMD 10, described above in relation to FIGS. 1 and 2. and power delivery status. Note that the examples shown in table 1 are just to show possible options for using indicator lights 64, and an audio output, to communicate with a user, and are not intended to limit pulse, flashing, color or other indications in any way.

**Table 1 - Indicator Light Functions**

| State | Function | Visual | Color | Sound | Haptic |
|---|---|---|---|---|---|
| UI-TEL | Trying to connect | Side-to-side | Green | | |
| UI-LOC | Poor Coupling | Spinning | Green | | YES |
| UI-CLL ON | Closed Loop Good Coupling | Pulsing | Green | | |
| UI-CP ON | Closed Loop Poor Coupling | Spinning | Green | Beep | YES |
| UI-CLL OFF | Closed Loop Good Coupling | Pulsing | Orange | | |
| UI-CP OFF | Closed Loop Poor Coupling | Spinning | Orange | Beep | YES |
| UI-OL (Patient) | Open Loop Good Coupling | Side-to-side | Green | | |
| UI-SPC | Physician Mode Searching | Side-to-side | Orange | | |
| UI-PRM | Physician Mode Charging | Pulsing with pause for N seconds | Orange | | |
| UI-CON | Connected | Solid | | | |
| UI-CC | Charge Complete | Solid | Green | | |

Processing circuitry 50 may, when requested, transmit any stored data in memory 52 to another computing device for review or further processing, such as to server 112 depicted in FIG. 1. Processing circuitry 50 may be configured to access memory, such as memory 32 of IMD 14 and/or memory 52 of external charging device 22, to retrieve information comprising instructions, formulas, and determined values for one or more constants.

User interface 54 may include buttons, such as control 62 or a keypad, lights, such as indicator lights 64, a speaker for voice commands, a display, such as a liquid crystal (LCD), light-emitting diode (LED), or cathode ray tube (CRT). In some examples, the display may be a touch screen. Control 62 may be implemented as any type of component that may receive user input and provide an indication of the user input to processing circuitry 50. As described above in relation to FIG. 1, control 62 may be a knob, switch, button and so on. As discussed in this disclosure, processing circuitry 50 may present and receive information relating to the charging of rechargeable power source 18 via user interface 54. For example, user interface 54 may indicate when charging is occurring, quality of the alignment between primary coil 28 or 48 and the secondary coil of the IMD. the selected power level, current charge level of rechargeable power source 18, duration of the current recharge session, anticipated remaining time of the charging session, sensed temperatures, or any other information. Processing circuitry 50 may receive some of the information displayed on user interface 54 from IMD 14 in some examples. In some examples, user interface 54 may provide an indication to the user regarding the quality of alignment between coil 16. depicted in FIG. 2 and coil 48, based on the charge current to the battery.

User interface 54 may also receive user input via user interface 54. The input may be, for example, in the form of pressing a button on a keypad or selecting an icon from a touch screen. The input may change programmed settings, start or stop therapy, request starting or stopping a recharge session, a desired level of charging, or one or more statistics related to charging rechargeable power source 18 (e.g., the cumulative thermal dose). In this manner, user interface 54 may allow the user to view information related to the operation of IMD 14. For example, control 62 may provide an input to processing circuitry 50 to cause processing circuitry 50 to start or stop delivery of wireless power to the power receiving device, e.g., IMD 10 described above in relation to FIGS. 1 and 2.

Charging circuitry 58 may include one or more circuits that generate an electrical signal, and an electrical current, within primary coil 48. Charging circuitry 58 may generate an alternating current of specified amplitude and frequency in some examples. In other examples, charging circuitry 58 may generate a direct current. In any case, charging circuitry 58 may be capable of generating electrical signals, and subsequent magnetic fields, to transmit various levels of power to IMD 14. In this manner, charging circuitry 58 may be configured to charge rechargeable power source 18 of IMD 14 with the selected power level.

Power source 60 may deliver operating power to the components of external charging device 22. Power source 60 may also deliver the operating power to drive primary coil 48 during the charging process. Power source 60 may include a battery and a power generation circuit to produce the operating power. In some examples, a battery of power source 60 may be rechargeable to allow extended portable operation. In other examples, power source 60 may draw power from a wired voltage source such as a consumer or commercial power outlet.

Telemetry circuitry 56 supports wireless communication between IMD 14 and external charging device 22 under the control of processing circuitry 50. Telemetry circuitry 56 may also be configured to communicate with another computing device via wireless communication techniques, or direct communication through a wired connection. In some examples, telemetry circuitry 56 may be substantially similar to telemetry circuitry 36 of IMD 14 described herein, providing wireless communication via an RF or proximal inductive medium. In some examples, telemetry circuitry 56 may include an antenna 57, which may take on a variety of forms, such as an internal or external antenna. Although telemetry modules 56 and 36 may each include dedicated antennas for communications between these devices, telemetry modules 56 and 36 may instead, or additionally, be configured to utilize inductive coupling from coils 16 and 48 to transfer data.

Examples of local wireless communication techniques that may be employed to facilitate communication between external charging device 22 and IMD 14 include radio frequency and/or inductive communication according to any of a variety of standard or proprietary telemetry protocols, or according to other telemetry protocols such as the IEEE 802.11x or Bluetooth specification sets. In this manner, other external devices may be capable of communicating with external charging device 22 without needing to establish a secure wireless connection.

In operation, processing circuitry 50, and or secondary processing circuitry 40, may control one or more sets of indicator lights 64 to provide information to a user about communication, charging efficiency, therapy status of the IMD and so on. For example, processing circuitry 50 may determine whether communication circuitry, e.g., telemetry circuitry 56, has established a communication link with the power receiving device, IMD 10 depicted in FIGS. 1 and 2. Responsive to determining that telemetry circuitry 56 has not established a communication link, processing circuitry 50 may cause at least two indicator lights of the set of indicator lights 64 to alternately flash.

As shown above in table 1, processing circuitry 50 may also determine whether the power receiving device is receiving wireless power, e.g., via charging circuitry 68 and coil 28, or charging circuitry 58 and coil 48. Responsive to determining that the power receiving device is receiving wireless power above a threshold, processing circuitry 50 may cause the set of indicator lights to pulse. As noted above in the description of FIG. 1, a pulse, or pulsing pattern means one or more indicator lights of the set of indicator lights 64 may increase in brightness followed by a decrease in brightness, e.g., fade on and off.

As described above in relation to FIG. 1, processing circuitry 50 may use any one or more system metrics to determine power transfer to IMD 10. In some examples, IMD 10 may send a signal indicating an amount of current output by the recharge circuitry of IMD 10. In other examples, processing circuitry 50 may calculate other system metrics, such as alignment of coil 28 to coil 16 of IMD 10 using any of several techniques, including heat calculations, temperature measurements, detection of metal, and so on. Processing circuitry may compare any of the calculated power transfer, power efficiency, alignment, IMD 10 current, etc. to a threshold stored at memory 52. When above the threshold, processing circuitry 50 may cause indicator lights 64 to pulse. Responsive to determining that the power receiving device is receiving power below the threshold, processing circuitry 50 may cause the set of indicator lights to output a spinning pattern. In some examples indicator lights 64 may illuminate control 62. In some examples, the spinning pattern may appear to circle control 62 in a clockwise or counter-clockwise direction.

Memory 52 may store several power coupling thresholds. A calculated power transfer metric (e.g., efficiency, magnitude of current, etc.) below a first threshold may indicate poor coupling. A power transfer metric above the first threshold but less than a second threshold may indicate "good" coupling. A power transfer metric above the second threshold but less than a third threshold may indicate "excellent" coupling, and so on. In some examples, responsive to determining that the power receiving device is receiving wireless power above the first threshold and receiving wireless power below the second threshold, processing circuitry 50 may cause the set of indicator lights 62, or one or more indicator lights of indicator lights 62, to pulse with a first pulsing pattern. Similarly, responsive to determining that the power receiving device is receiving wireless power above the second threshold, and receiving wireless power below the third threshold, processing circuitry 50 may cause the set of indicator lights 62 to pulse with a second pulsing pattern. In this disclosure, reference to actions of indicator lights 62 may include the entire set of indicator lights, one indicator light, or a portion of the indicator lights 62 pulsing, flashing, occulting, and so on in any combination.

In some examples, processing circuitry 50 may determine a therapy delivery state of the implantable medical device. A patient, or caregiver, may turn on or off the therapy delivery, e.g., electrical stimulation pulses, drug delivery etc. using an external computing device, such as a programmer, tablet computer and so on. Responsive to determining that the therapy delivery state is ON, processing circuitry 50 of recharger 22 may cause the indicator lights to output a first color, such as green, blue, amber, some other color or combination of colors. Responsive to determining that the therapy delivery state is OFF, cause the indicator lights to output a second color. In other examples, processing circuitry 50 may cause one or more indicator lights to flash or pulse in a predefined pattern based on the therapy delivery state.

In some examples, processing circuitry 50 may control haptic output circuitry 72 to provide a tactile sensation above the patient s perception level. For example, haptic output circuitry may vibrate or provide some similar tactile sensation. In some examples processing circuitry 50 may control haptic output circuitry to vibrate at a constant level for a specified duration, may output a pattern of vibration, or some similar haptic feedback for the patient. In some examples, the haptic feedback may indicate poor coupling, and the haptic feedback may fade as the coupling improves, e.g., the power receiving device is receiving wireless power above the first threshold. In this manner, the patient may receive feedback without the need to view user interface 54 of external charging device 22, or the user interface of some other device, e.g., a smart phone, tablet and so on.

In some examples, processing circuitry 50 may also output an audio tone, pattern, and so on via audio output circuitry 70. In some examples, the audio pattern may be selectable by a user, such as the patient, based on the type of information that processing circuitry 50 is programmed to output. Some examples of patterns may include a warble tone, a distinctive musical sequence (e.g.. shave and a haircut, Smoke on the Water intro, etc.) and so on. For example, responsive to determining that the power receiving device is receiving wireless power above the first threshold and receiving wireless power below a second threshold, processing circuitry 50 may cause audio circuitry to output an audible alert with a first audible pattern. Processing circuitry 50 may cause audio output circuitry 70 to output other audio tones, patterns and so on for any of the conditions or states described herein, e.g., therapy on or off, and so on.

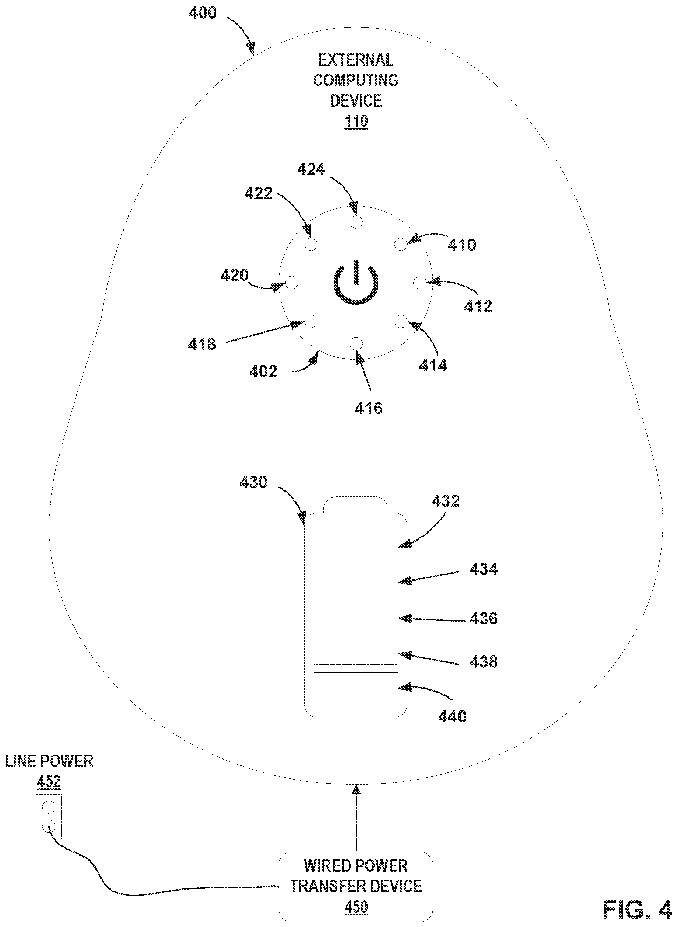

FIG. 4 is a conceptual diagram illustrating an example user interface for an external charging device, according to one or more techniques of this disclosure. External computing device 110 in the example of FIG. 4 is an example of external computing device 110 described above in relation to FIG. 1 and external charging device 22 described above in relation to FIG. 3, and may have the same or similar functions and characteristics.

External computing device 110, in the example of FIG. 4, includes housing 400, button 402 and battery indicator 430. External computing device 110 may include a rechargeable battery, which may be charged by connecting to wired power transfer device 450. Wired power transfer device 450 may receive power from line power 452, or some other power source, such as a generator. battery, solar cell. and so on. Wired power transfer device 450 may include electrical, as well as mechanical connections to external computing device 110. In this disclosure wired power transfer device 450 may also be referred to as dock 450. External computing device 110 may be considered "docked" when connected to wired power transfer device 450 and receiving power. External computing device 110 may also include one or more power transfer and/or communication antennae (not shown in FIG. 4), similar to coil 28 described above in relation to FIGS. 1 and 3.

As described above in relation to FIGS. 1 and 3, external computing device 110, also called recharger 110, may include a set of indicator lights 410 - 424, which may be configured to illuminate button 402 and output information regarding communication status and wireless power transfer status. In the example of FIG. 4, button 402 is depicted as a power button. In other examples button 402 may perform other functions. In other examples, recharger 110 may have additional buttons not shown in FIG. 4.

Processing circuitry of recharger 110 may determine whether communication circuitry, e.g., telemeter circuitry 56 depicted in FIG. 3, has established a communication link with the power receiving device. Responsive to determining that the communication circuitry has not established a communication link, the processing circuitry may cause at least two indicator lights of the set of indicator lights to alternately flash. For example, light 420 may alternately flash with light 412. In other examples, multiple lights may flash. or output other light patterns. For example, lights 410 and 412 may alternately flash with lights 418 and 420.

In some examples recharger 110 may still transcutaneously transfer power to the power receiving device without establishing communication with the power receiving device. In other words recharger 110 may operate in an open loop charging mode. Recharger 110 may indicate open loop charging by changing the pattern of the alternating indicator lights to pulsing and/or by changing the color of the indicator lights. For example, responsive to determining that the power receiving device is receiving wireless power above a threshold. the processing circuitry may cause indicator lights 412 and 420 to alternately pulse, as noted above in table 1.

The processing circuitry may display closed loop charging, such as when communication circuitry establishes a communication link, by one or more indicator lights pulsing. In some examples, all the indicator lights that illuminate button 402 may pulse to signify closed loop charging, e.g., above a predetermined threshold. For both open loop and closed loop charging, changes in the pulsing pattern or color may indicate different coupling levels, as described above in relation to FIG. 1.

Responsive to determining that the power receiving device is receiving power below the threshold, e.g., poor coupling, the processing circuitry may cause the set of indicator lights to output a spinning pattern. For example, light 420 may turn on and begin to fade off, followed by light 422 increasing in brightness, then beginning to fade off, followed by light 424 increasing in brightness, then beginning to fade off, and so on, which may give the impression that the set of indicator lights is spinning around button 402.

The processing circuitry may also determine a therapy delivery state of the implantable medical device during recharging. In some examples, responsive to determining that the therapy delivery state is ON, the processing circuitry may cause the indicator lights to output a first color, such as green, blue, and so on. The color may be independent of the coupling state and communication state. For example, responsive to determining that the therapy delivery state is OFF, and that the implantable medical device is receiving wireless power above a threshold, the processing circuitry may cause the indicator lights to output a second color, e.g., amber, orange, or some other color in a pulsing pattern. The pulsing pattern may indicate the coupling level, as described above in relation to FIG. 1. Similarly, a poor power transfer coupling, may result in a spinning pattern in a first color, with therapy ON and in a second color, with therapy OFF.

Battery indicator 430 may include a second set of indicator lights 432 - 440. which are separate from the indicator lights that illuminate button 402. The second set of indicator lights 432 - 440 may provide an indication of a charge level for a first electrical storage unit, such as a battery of the power transmitting device, recharger 110. For example, the processing circuitry may signify a full charge of the battery for recharger 110 by lighting all of lights 440, 438, 436, 434 and 432. In some examples, while connected to dock 450, and connected to line power 452, the second set of indicator lights 432 - 440 may light in a flashing or occulting pattern to indicate that recharger 110 is being charge.

At a different time the second set of indicator lights 432 - 440 may provide an indication of a charge level for the electrical storage unit, e.g., the battery of the power receiving device, for example, IMD 10 depicted in FIGS. 1 and 2. For example, when disconnected from dock 450 and in a communication session with IMD 10, recharger 110 may receive a signal from IMD 10 indicating the state of charge of the battery for IMD 10. For example lights 440 and 438 may light when the charge level for the IMD 10 is less than half and more lights may indicate a higher charge level.

In some examples, indicator lights 432 - 440 may indicate the charge level for the power receiving device battery with a first color, e.g., orange, amber and so on, and indicate the charge level for recharger 110 with a second color, e.g., green, blue and so on. In some examples, the second set of indicator lights 432 - 440 may provide the indication of the charge for the first electrical storage unit of the power transmitting device when the power transmitting device is electrically connected to dock 450 at a first time. The second set of indicator lights of battery indicator 430 may signify the charge for the battery of the power receiving device when recharger 110 is disconnected from the wired power transfer device. In some examples, one or more of indicator lights 432 - 440 may display the battery of recharger 110 does not have enough energy to charge IMD 10. For example, the lowest battery bar, light 440, may be amber, red or some other color, when recharger 110 is connected to dock 450, but there is not enough energy to charge the IMD 10. In other examples, the lowest battery bar, e.g., light 440, or light 440 in combination with other lights on battery indicator 430 when in an open loop charging session and when in a closed loop charging session. For example, light 440 may be colored amber when in open loop recharge mode (no telemetry with the IMD 10) and a different color when in closed loop recharge mode. In other examples, one or more of indicator lights 432 - 440 may flash, pulse or otherwise indicate a difference between open loop and closed loop charging.

The user interface of the external computing device 110 may have advantages over other types of user interfaces. By using only a few indicator lights, and taking advantage of the various colors, flashing, pulsing and other patterns described above, may provide a user a convenient way to receive feedback for positioning for efficient power transfer. In addition, the user may be able to quickly determine the status of the IMD, e.g., therapy ON or therapy OFF, communication status, battery charge level, and so on.

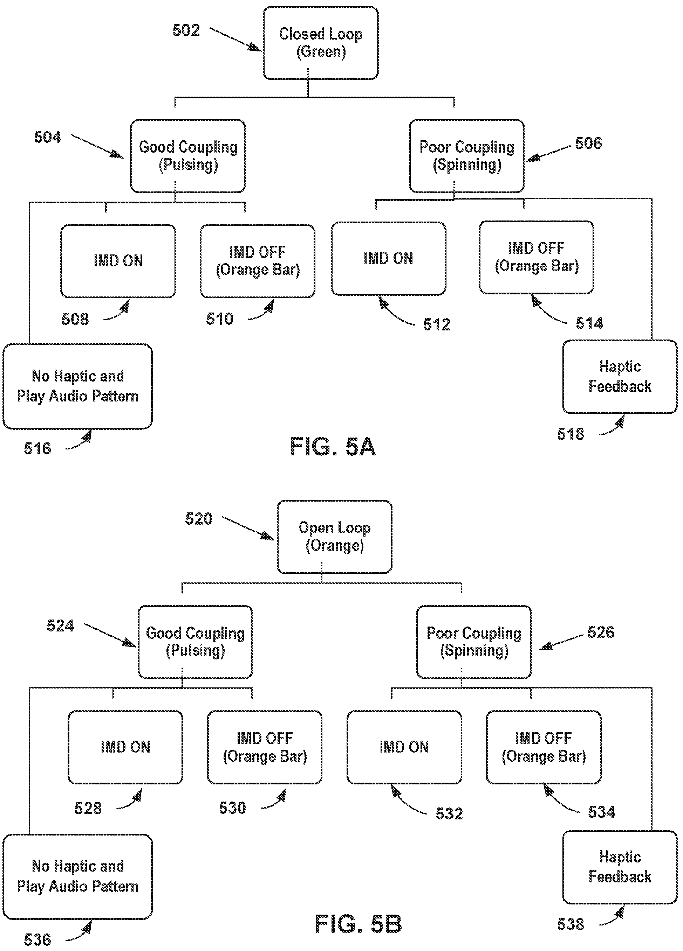

FIGS. 5A and 5B are tree charts illustrating examples of indications from the user interface of the external charging device according to one or more techniques of this disclosure. The examples illustrated in FIGS. 5A and 5B apply to the user interface described above in relation to FIG. 4.

FIG. 5A illustrates an example of the user interface while an external computing device is transferring electrical energy to an implantable medical device, e.g., external computing device 110 transferring power to IMD 10 as described above in relation to FIG. 1, while in communication with IMD 10. Charging while communicating with the IMD may be considered closed loop charging 502. One or more of indicator light 410 - 424 on button 402 may be illuminated with a first color, e.g., green, as shown in the example of FIG. 5A. The first color, or the second color may be green or any other color that an RGB or similar LED may produce.

When the relative position of the recharger coil and the secondary coil of the IMD is such that power is being transferred with good coupling, e.g., above a threshold as described above in relation to FIG.1, one or more of indicator lights 410 - 424 may be pulsing 504. With poor coupling, e.g., a power transfer level below a power transfer threshold, indicator lights 410 - 424 may sequentially flash to give the appearance the indicator lights on button 402 are spinning 506, e.g., either clockwise or counter-clockwise.

One or more of indicator lights 432 - 440 on battery indicator 430 may output an indication that the IMD is ON (508, 512, 528 and 532), or the IMD is OFF (510, 514, 530 and 534). In some examples indicator lights 432 - 440 on battery indicator 430 may be illuminated in a first color, e.g., green, when the IMD is ON and delivering therapy. The indicator lights may be illuminated in a second color, e.g., orange, as shown in the example of FIGS. 5A and 5B, when the IMD is OFF and not delivering therapy.

Similarly, when the external computing device delivers wireless electrical energy, but is unable to communicate with the IMD, one or more of indicator light 410 - 424 on button 402 may be illuminated with a second color, e.g., orange, as shown in the example of FIG. 5B. Though unable to communicate with the IMD, the external charging device may determine the level of power transfer using any of several techniques, including heat calculations, temperature measurements, detection of metal, and so on, as described above in relation to FIG. 1. As described above in relation to FIG. 5A, with good coupling, one or more of indicator lights 410 - 424 may be pulsing 524. With poor coupling, e.g.. a power transfer level below a power transfer threshold, indicator lights 410 - 424 may sequentially flash to give the appearance the indicator lights on button 402 are spinning 526.

As described above in relation to FIG. 3, in some examples, the user interface may include an audio output as well as a haptic output. In the example of FIGS. 5A and 5B, with poor coupling (506, 526), e.g., wireless power transfer below a predetermined threshold, the processing circuitry may cause the haptic output circuitry to provide a vibration or some other tactile sensation to the patient (518, 530). With good coupling between the external charging device and the power receiving device (504, 524). e.g., power transfer or power transfer efficiency above a predetermined threshold, the haptic feedback may fade and/or disappear (516, 536). In some examples, the processing circuitry may also cause the audio output circuit to play a tone or pattern that indicates good power transfer (516, 536). In this manner, the external charging device of this disclosure may provide the user with a variety of visual, tactile and audio means to know how the recharger coupling is performing.

As noted above, the example of FIGS. 5A and 5B is just one possible implementation to provide user feedback. In other examples, the external charging device may vibrate or otherwise output haptic feedback to show good coupling, may output a first haptic pattern for good coupling and a second pattern for poor coupling, and so on. Similarly, the audio output circuitry, may play a first tonal pattern for good coupling, a second for poor coupling and, and along with the haptic output circuitry, output other patterns to indicate communication status, IMD ON or OFF status and so on.

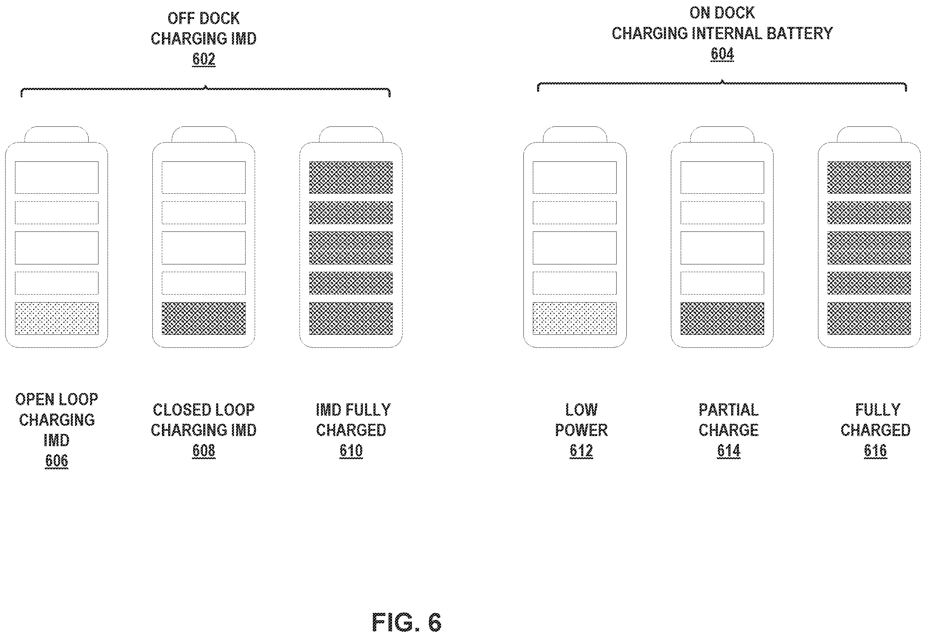

**FIG.** 6 is a conceptual diagram illustrating example indications from the user interface based the status of the external charging device of this disclosure. The examples illustrated in FIG. 6 apply to the user interface described above in relation to FIG. 4. The example of FIG. 6 describes an alternative operation to the indications described above in relation to FIGS. 5A and 5B.

While charging the IMD, and not connected 602 to a wired power transfer device, e.g., wired power transfer device 450 described above in relation to FIG. 4. the indicator lights of the battery indicator, e.g., battery indicator 430 may output an indication of the charging level of the IMD. e.g., fully charged 610 or less than fully charged as shown in 606 and 608. In this disclosure, a wired power transfer device, e.g., wired power transfer device 450 may be configured as a docking station, e.g., a dock. The external computing device may be ON the dock and receiving power 604, or OFF the dock 602. e.g.. while delivering wireless power to the IMD.

In the example in which the external computing device, e.g., external computing device 110 is not in communication with IMD 10 as described above in relation to FIG. 1. the external computing device may deliver electrical energy in an open loop mode, and indicate open loop charging 606 with a selected color for the one or more of indicator lights 432 - 440 on battery indicator 430, depicted in FIG. 4. For example, the one or more indicator lights may be orange, amber or some other color.

When the external computing device is able to establish communication with the IMD, the one or more of indicator lights may output a different color, e.g., blue, green and so on (608, 610). As the IMD battery changes, additional indicator lights may illuminate until the IMD battery is fully charged 610.

When connected to the wired power transfer device. e.g., on the dock 604 and charging the internal battery of the external computing device, the battery indicator may output the status of internal battery of the external computing device. For example, when the battery level is low. e.g.. below a threshold charging level, the battery indicator may illuminate one or more of the indicator lights 612 with one color. e.g., amber, yellow, red and so on. In some examples, when the internal battery charging level is too low to deliver a complete charge to an IMD, the processing circuitry of the external computing device may cause the indicator lights to output an indication of low power 612.

While charging, and the processing circuitry of the external computing device determines that the battery charge level is above a predetermined threshold, e.g., enough power to recharge an IMD, the processing circuitry may cause one or more indicator lights to change to a different color, e.g., green, blue, and so on to indicate a partial charge 614. When fully charged 616, the indicator lights may all be illuminated. In some examples, the fully charged color 616, may be the same as the color for partial charge 614.

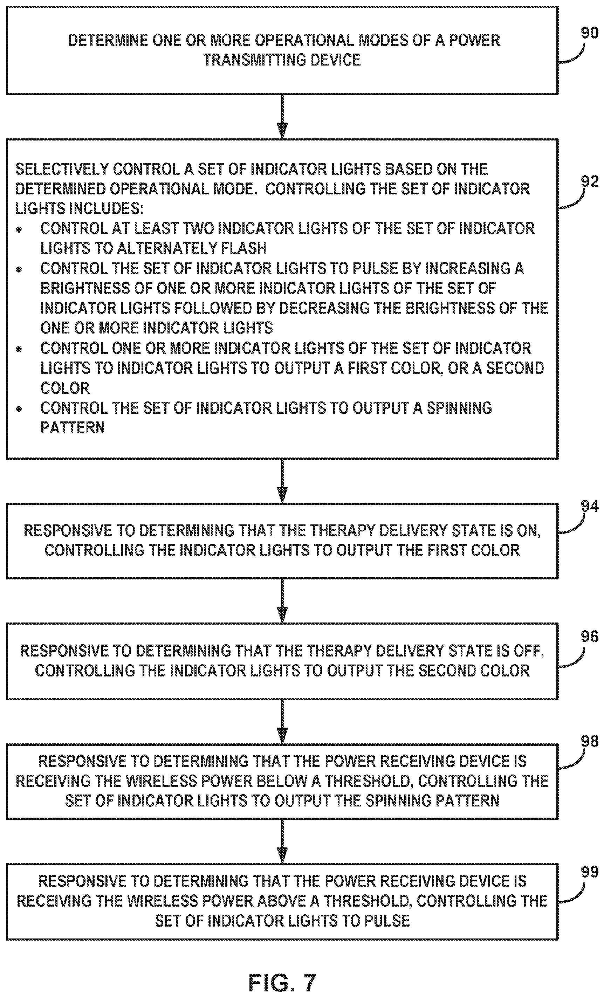

FIG. 7 is a flow chart illustrating an example operation of an external charging device according to one or more techniques of this disclosure. Processing circuitry of a power transmitting device, e.g., external computing device 110 and external computing device 22 described above in relation to FIGS. 1 and 3 respectively, may determine whether communication circuitry of the power transmitting device has established a communication link with a power receiving device (90). The power receiving device may be a medical device, such as an implantable medical device, e.g., IMD 10 and IMD 14 described above in relation to FIGS. 1 and 2 respectively.

Responsive to determining that the communication circuitry. e.g., telemetry circuitry 56 depicted in FIG. 3 has not established a communication link, the processing circuitry, e.g., processing circuitry 50 of the power transmitting device may cause at least two indicator lights of a set of indicator lights to alternately flash (92). For example, as described above in relation to FIG. 4, on button 402, light 420 may alternately flash with light 412 or multiple lights may flash to indicate that the communication circuitry has not established a communication link.

The processing circuitry of the wireless power transfer device may further, determine whether the power receiving device is receiving wireless power (94). Responsive to determining that the power receiving device is receiving wireless power above a threshold, the processing circuitry may cause the set of indicator lights 410 - 424 of button 402 to pulse (96). As described above in relation to FIG. 1, a pulsing indication is when one or more indicator lights of the set of indicator lights will increase in brightness followed by a decrease in brightness.

In one or more examples, the functions described above may be implemented in hardware, software, firmware, or any combination thereof. For example, the various components of FIGS. 2 and 3, such as processing circuitry 30 and processing circuitry 50 may be implemented in hardware, software, firmware, or any combination thereof. If implemented in software, the functions may be stored on or transmitted over, as one or more instructions or code, a computer-readable medium and executed by a hardware-based processing unit.

Computer-readable media, e.g., memory 32 and memory 52 depicted in FIGS. 2 and 3, may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

The term "non-transitory" may indicate that the storage medium is not embodied in a carrier wave or a propagated signal. In certain examples, a non-transitory storage medium may store data that can, over time, change (e.g., in RAM or cache). By way of example, and not limitation, such computer-readable storage media, may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or other computer readable media. In some examples, an article of manufacture may include one or more computer-readable storage media.

Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transient media, but are instead directed to non-transient, tangible storage media. Combinations of the above should also be included within the scope of computer-readable media.

Instructions may be executed by one or more processors, such as one or more DSPs, general purpose microprocessors, ASICs, FPGAs, or other equivalent integrated or discrete logic circuitry. Accordingly, the term "processor," as used herein, may refer to any of the foregoing structure or any other structure suitable for implementation of the techniques described herein. Also, the techniques could be fully implemented in one or more circuits or logic elements.

The techniques of this disclosure may be implemented in a wide variety of devices or apparatuses, including, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

The scope of the invention is defined by the following claims.

## Claims

1. A power transmitting device configured to wirelessly transfer power to a power receiving device (14), wherein the power receiving device (14) is an implantable medical device (14) having a rechargeable power source and configured to deliver a therapy to a patient, the power transmitting device (22) comprising:
a user interface (54) including:
a control configured to receive user input; and
a set of indicator lights configured to output information regarding communication status and wireless power transfer status;
circuitry (56, 68) configured to:
wirelessly communicate with the power receiving device (14); and
wirelessly output power to the power receiving device (14) to recharge the rechargeable power source of the implantable medical device;
processing circuitry (50) operably coupled to a memory (52), the processing circuitry configured (50) to:
determine one or more operational states, wherein the one or more operational states comprise:
a therapy delivery state of the implantable medical device (14);
selectively control, based on the operational state, the set of indicator lights (64) to perform an action, wherein the action comprises:
responsive to determining that the therapy delivery state is ON, control the indicator lights to output a first color, and
responsive to determining that the therapy delivery state is OFF, control the indicator lights to output a second color.

2. The power transmitting device of claim 1, wherein the one or more operational states further comprise at least one of:
that the communication circuitry (56) has not established a communication link with the power receiving device (14); or
that the power receiving device (14) is receiving wireless power above a threshold, and
wherein the processing circuitry (50) is further configured to:
selectively control, based on the operational state, the set of indicator lights (64) to perform an action, wherein the action comprises:
controlling at least two indicator lights (64) of the set of indicator lights to alternately flash;
controlling the set of indicator lights (64) to pulse by increasing a brightness of one or more indicator lights (64) of the set of indicator lights followed by decreasing the brightness of the one or more indicator lights (64);
controlling the one or more indicator lights (64) of the set of indicator lights to output one of a first color or a second color; and
controlling the set of indicator lights to output a spinning pattern.

3. The power transmitting device of claim 2, wherein responsive to determining that the circuitry has not established the communication link, the processing circuitry is configured to control at least two indicator lights of the set of indicator lights to alternately flash.

4. The power transmitting device of any of claims 1 - 3,
wherein the control of the user interface comprises at least one of: a button, a switch, and a knob; and
wherein the indicator lights are configured to illuminate the control.

5. The power transmitting device of any of claims 2 - 4,
wherein the one or more operational states comprise that the power receiving device is receiving power below the threshold; and
wherein responsive to determining that the power receiving device is receiving power below the threshold, the processing circuitry is configured to control the set of indicator lights to output the spinning pattern.

6. The power transmitting device of any of claims 2 - 5, wherein the threshold is a first threshold, and wherein the processing circuitry is further configured to:
responsive to determining that the power receiving device is receiving wireless power above the first threshold and receiving wireless power below a second threshold, cause the set of indicator lights to pulse with a first pulsing pattern, and
responsive to determining that the power receiving device is receiving wireless power above the second threshold, and receiving wireless power below a third threshold, cause the set of indicator lights to pulse with a second pulsing pattern.

7. The power transmitting device of any of claims 2 - 6, wherein the processing circuitry is further configured to, responsive to determining that the power receiving device is receiving wireless power above the first threshold and receiving wireless power below a second threshold, cause audio circuitry to output an audible alert with a first audible pattern.

8. The power transmitting device of any of claim 4, wherein the set of indicator lights configured to illuminate the control is a first set of indicator lights, the power transmitting device further comprising a second set of indicator lights separate from the button, wherein the second set of indicator lights provides:
an indication of a charge level for a first electrical storage device of the power transmitting device at a first time; and
an indication of a charge level for a second electrical storage device of the power receiving device at a second time.

9. The power transmitting device of any of claim 8, wherein the processing circuitry is further configured to cause an indicator light of the second set of indicators lights to output an indication that the first electrical storage device for the power transmitting device has insufficient electrical energy available to deliver wireless power to the power receiving device.

10. The power transmitting device of any of claims 2 - 9, wherein responsive to determining that the power receiving device is receiving wireless power above the threshold, the processing circuitry is configured to control the set of indicator lights to pulse.

11. A system comprising:
the power transmitting device of any of the claims 1 to 10, and
the power receiving device.

12. The system of claim 11, further comprising an external computing device configured to wirelessly communicate with the power transmitting device and the power receiving device, the external computing device comprising a touch-screen user interface.

13. The system of claims 11 and 12, further comprising a wired power transfer device configured to transfer electrical power to the power transmitting device.

14. The system of claim 13, wherein the set of indicator lights is a first set of indicator lights, the power transmitting device further comprising a second set of indicator lights, wherein the second set of indicator lights provides:
an indication of a charge level for a first electrical storage unit of the power transmitting device at a first time; and
an indication of a charge level for a second electrical storage unit of the power receiving device at a second time.

15. The system of claim 14, wherein the second set of indicator lights provides:
the indication of the charge level for the first electrical storage unit of the power transmitting device when the power transmitting device is electrically connected to the wired power transfer device; and
the indication of the charge level for the second electrical storage unit of the power receiving device when the power transmitting device is disconnected from the wired power transfer device.

## Patentansprüche

1. Leistungsübertragungsvorrichtung, die konfiguriert ist, um Leistung drahtlos an eine Leistungsempfangsvorrichtung (14) zu transferieren, wobei die Leistungsempfangsvorrichtung (14) eine implantierbare medizinische Vorrichtung (14) ist, die eine wiederaufladbare Leistungsquelle aufweist und konfiguriert ist, um eine Therapie an einen Patienten abzugeben, wobei die Leistungsübertragungsvorrichtung (22) Folgendes umfasst:
eine Benutzerschnittstelle (54), die Folgendes einschließt:
eine Steuerung, die konfiguriert ist, um eine Benutzereingabe zu empfangen; und
einen Satz von Anzeigelichtern, die konfiguriert sind, um Informationen bezüglich eines Kommunikationsstatus und eines Status eines drahtlosen Leistungstransfers auszugeben;
eine Schaltung (56, 68), die konfiguriert ist, um:
drahtlos mit der Leistungsempfangsvorrichtung (14) zu kommunizieren; und
drahtlos Leistung an die Leistungsempfangsvorrichtung (14) auszugeben, um die wiederaufladbare Leistungsquelle der implantierbaren medizinischen Vorrichtung wieder aufzuladen;
eine Verarbeitungsschaltung (50), die mit einem Speicher (52) betriebsfähig gekoppelt ist, wobei die Verarbeitungsschaltung (50) konfiguriert ist, um:
einen oder mehrere Betriebszustände zu bestimmen, wobei der eine oder die mehreren Betriebszustände Folgendes umfassen:
einen Therapieabgabezustand der implantierbaren medizinischen Vorrichtung (14); auf Basis des Betriebszustands den Satz von Anzeigelichtern (64) selektiv zu steuern, um eine Aktion durchzuführen, wobei die Aktion Folgendes umfasst:
als Reaktion auf das Bestimmen, dass der Therapieabgabestatus EIN ist, die Anzeigelichter zu steuern, um eine erste Farbe auszugeben, und
als Reaktion auf das Bestimmen, dass der Therapieabgabezustand AUS ist, die Anzeigelichter zu steuern, um eine zweite Farbe auszugeben.

2. Leistungsübertragungsvorrichtung nach Anspruch 1, wobei der eine oder die mehreren Betriebszustände ferner mindestens eines aus Folgendem umfassen:
dass die Kommunikationsschaltung (56) keine Kommunikationsverknüpfung mit der Leistungsempfangsvorrichtung (14) hergestellt hat; oder
dass die Leistungsempfangsvorrichtung (14) drahtlose Leistung oberhalb eines Schwellenwerts empfängt, und
wobei die Verarbeitungsschaltung (50) ferner konfiguriert ist um:
auf Basis des Betriebszustands den Satz von Anzeigelichtern (64) selektiv zu steuern, um eine Aktion durchzuführen, wobei die Aktion Folgendes umfasst:
Steuern von mindestens zwei Anzeigelichtern (64) des Satzes von Anzeigelichtern, um abwechselnd zu blinken;
Steuern des Satzes von Anzeigelichtern (64), um zu pulsieren, durch Erhöhen einer Helligkeit eines oder mehrerer Anzeigelichter (64) des Satzes von Anzeigelichtern, gefolgt von Verringern der Helligkeit des einen oder der mehreren Anzeigelichter (64);
Steuern der einen oder mehreren Anzeigelichter (64) des Satzes von Anzeigelichtern, um eine erste Farbe oder eine zweite Farbe auszugeben; und
Steuern des Satzes von Anzeigelichtern, um ein sich drehendes Muster auszugeben.

3. Leistungsübertragungsvorrichtung nach Anspruch 2, wobei die Schaltung als Reaktion auf das Bestimmen, dass die Schaltung die Kommunikationsverknüpfung nicht hergestellt hat, konfiguriert ist, um mindestens zwei Anzeigelichter des Satzes von Anzeigelichtern zu steuern, um abwechselnd zu blinken.

4. Leistungsübertragungsvorrichtung nach einem der Ansprüche 1 bis 3,
wobei die Steuerung der Benutzerschnittstelle mindestens eines von Folgendem umfasst: eine Taste, einen Schalter und einen Drehknopf; und
wobei die Anzeigelichter konfiguriert sind, um die Steuerung zu beleuchten.

5. Leistungsübertragungsvorrichtung nach einem der Ansprüche 2 bis 4,
wobei der eine oder die mehreren Betriebszustände umfassen, dass die Leistungsempfangsvorrichtung Leistung unterhalb des Schwellenwerts empfängt; und
wobei die Verarbeitungsschaltung als Reaktion auf das Bestimmen, dass die Leistungsempfangsvorrichtung Leistung unterhalb des Schwellenwerts empfängt, konfiguriert ist, um den Satz von Anzeigelichtern zu steuern, um das sich drehende Muster auszugeben.

6. Leistungsübertragungsvorrichtung nach einem der Ansprüche 2 bis 5, wobei der Schwellenwert ein erster Schwellenwert ist, und wobei die Verarbeitungsschaltung ferner konfiguriert ist, um:
als Reaktion auf das Bestimmen, dass die Leistungsempfangsvorrichtung drahtlose Leistung oberhalb des ersten Schwellenwerts empfängt und drahtlose Leistung unterhalb eines zweiten Schwellenwerts empfängt, zu veranlassen, dass der Satz von Anzeigelichtern mit einem ersten Pulsmuster pulsiert, und
als Reaktion auf das Bestimmen, dass die Leistungsempfangsvorrichtung drahtlose Leistung oberhalb des zweiten Schwellenwerts empfängt und drahtlose Leistung unterhalb eines dritten Schwellenwerts empfängt, zu veranlassen, dass der Satz von Anzeigelichtern mit einem zweiten Pulsmuster pulsiert.

7. Leistungsübertragungsvorrichtung nach einem der Ansprüche 2 bis 6, wobei die Verarbeitungsschaltung ferner konfiguriert ist, um als Reaktion auf das Bestimmen, dass die Leistungsempfangsvorrichtung drahtlose Leistung oberhalb des ersten Schwellenwerts empfängt und drahtlose Leistung unterhalb eines zweiten Schwellenwerts empfängt, eine Audioschaltung zu veranlassen, einen hörbaren Alarm mit einem ersten hörbaren Muster auszugeben.

8. Leistungsübertragungsvorrichtung nach einem der Ansprüche 4, wobei der Satz von Anzeigelichtern, der konfiguriert ist, um die Steuerung zu beleuchten, ein erster Satz von Anzeigelichtern ist, wobei die Leistungsübertragungsvorrichtung ferner einen zweiten Satz von Anzeigelichtern umfasst, der von der Taste getrennt ist, wobei der zweite Satz von Anzeigelichtern Folgendes bereitstellt:
eine Anzeige eines Ladestands für eine erste elektrische Speichervorrichtung der Leistungsübertragungsvorrichtung zu einem ersten Zeitpunkt; und
eine Anzeige eines Ladestands für eine zweite elektrische Speichervorrichtung der Leistungsempfangsvorrichtung zu einem zweiten Zeitpunkt.

9. Leistungsübertragungsvorrichtung nach einem der Ansprüche 8, wobei die Verarbeitungsschaltung ferner konfiguriert ist, um zu veranlassen, dass ein Anzeigelicht des zweiten Satzes von Anzeigelichtern eine Anzeige ausgibt, dass die erste elektrische Speichervorrichtung für die Leistungsübertragungsvorrichtung unzureichende elektrische Energie verfügbar hat, um drahtlose Leistung an die Leistungsempfangsvorrichtung abzugeben.

10. Leistungsübertragungsvorrichtung nach einem der Ansprüche 2 bis 9, wobei als Reaktion auf das Bestimmen, dass die Leistungsempfangsvorrichtung drahtlose Leistung oberhalb des Schwellenwerts empfängt, die Verarbeitungsschaltung konfiguriert ist, um den Satz von Anzeigelichtern zu steuern, um zu pulsieren.

11. System, umfassend:
die Leistungsübertragungsvorrichtung nach einem der Ansprüche 1 bis 10; und
die Leistungsempfangsvorrichtung.

12. System nach Anspruch 11, ferner umfassend eine externe Rechenvorrichtung, die konfiguriert ist, um drahtlos mit der Leistungsübertragungsvorrichtung und der Leistungsempfangsvorrichtung zu kommunizieren, wobei die externe Rechenvorrichtung eine Touchscreen-Benutzerschnittstelle umfasst.

13. System nach Ansprüchen 11 und 12, ferner umfassend eine drahtgebundene Leistungstransfervorrichtung, die konfiguriert ist, um elektrische Leistung an die Leistungsübertragungsvorrichtung zu transferieren.

14. System nach Anspruch 13, wobei der Satz von Anzeigelichtern ein erster Satz von Anzeigelichtern ist, wobei die Leistungsübertragungsvorrichtung ferner einen zweiten Satz von Anzeigelichtern umfasst, wobei der zweite Satz von Anzeigelichtern Folgendes bereitstellt:
eine Anzeige eines Ladestands für eine erste elektrische Speichereinheit der Leistungsübertragungsvorrichtung zu einem ersten Zeitpunkt; und
eine Anzeige eines Ladestands für eine zweite elektrische Speichereinheit der Leistungsempfangsvorrichtung zu einem zweiten Zeitpunkt.

15. System nach Anspruch 14, wobei der zweite Satz von Anzeigelichtern Folgendes bereitstellt:
die Anzeige des Ladestands für die erste elektrische Speichereinheit der Leistungsübertragungsvorrichtung, wenn die Leistungsübertragungsvorrichtung elektrisch mit der drahtgebundenen Leistungstransfervorrichtung verbunden ist; und
die Anzeige des Ladestands für die zweite elektrische Speichereinheit der Leistungsempfangsvorrichtung, wenn die Leistungsübertragungsvorrichtung von der drahtgebundenen Leistungstransfervorrichtung getrennt ist.

## Revendications

1. Dispositif de transmission d'énergie configuré pour transférer sans fil de l'énergie à un dispositif de réception d'énergie (14), dans lequel le dispositif de réception d'énergie (14) est un dispositif médical implantable (14) ayant une source d'énergie rechargeable et configuré pour administrer une thérapie à un patient, le dispositif de transmission d'énergie (22) comprenant :
une interface utilisateur (54) comportant :
une commande configurée pour recevoir une entrée utilisateur ; et
un ensemble de voyants lumineux configurés pour émettre en sortie des informations concernant un état de communication et un état de transfert d'énergie sans fil ;
un circuit (56, 68) configuré pour :
communiquer sans fil avec le dispositif de réception d'énergie (14) ; et
fournir sans fil de l'énergie au dispositif de réception d'énergie (14) afin de recharger la source d'énergie rechargeable du dispositif médical implantable ;
un circuit de traitement (50) couplé de manière fonctionnelle à une mémoire (52), le circuit de traitement (50) étant configuré pour :
déterminer un ou plusieurs états de fonctionnement, dans lequel le ou les états de fonctionnement comprennent :
un état d'administration de thérapie du dispositif médical implantable (14) ; commander sélectivement, en fonction de l'état de fonctionnement, l'ensemble de voyants lumineux (64) pour effectuer une action, dans lequel l'action comprend :
en réponse à la détermination que l'état d'administration de thérapie est ACTIVÉ, commander les voyants lumineux pour qu'ils émettent en sortie une première couleur, et
en réponse à la détermination que l'état d'administration de thérapie est DÉSACTIVÉ, commander les voyants lumineux pour qu'ils émettent en sortie une seconde couleur.

2. Dispositif de transmission d'énergie selon la revendication 1, dans lequel le ou les états de fonctionnement comprennent en outre au moins l'un parmi :
le fait que le circuit de communication (56) n'a pas établi de liaison de communication avec le dispositif de réception d'énergie (14) ; ou
le fait que le dispositif de réception d'énergie (14) reçoit une énergie sans fil supérieure à un seuil, et
dans lequel, le circuit de traitement (50) est en outre configuré pour :
commander sélectivement, en fonction de l'état de fonctionnement, l'ensemble de voyants lumineux (64) pour effectuer une action, dans lequel l'action comprend :
la commande d'au moins deux voyants lumineux (64) de l'ensemble de voyants lumineux pour qu'ils clignotent alternativement ;
la commande de l'ensemble de voyants lumineux (64) pour qu'ils émettent une impulsion en augmentant une luminosité d'un ou plusieurs voyants lumineux (64) de l'ensemble de voyants lumineux, puis en diminuant la luminosité du ou des voyants lumineux (64) ;
la commande du ou des voyants lumineux (64) de l'ensemble de voyants lumineux pour qu'ils émettent en sortie l'une parmi une première couleur ou une seconde couleur ; et
la commande de l'ensemble de voyants lumineux pour qu'ils émettent en sortie un motif tournoyant.

3. Dispositif de transmission d'énergie selon la revendication 2, dans lequel, en réponse à la détermination du fait que le circuit n'a pas établi la liaison de communication, le circuit de traitement est configuré pour commander au moins deux voyants lumineux de l'ensemble de voyants lumineux pour qu'ils clignotent alternativement.

4. Dispositif de transmission d'énergie selon l'une quelconque des revendications 1 - 3,
dans lequel la commande de l'interface utilisateur comprend au moins l'un parmi : une touche, un interrupteur et un bouton ; et
dans lequel les voyants lumineux sont configurés pour éclairer la commande.

5. Dispositif de transmission d'énergie selon l'une quelconque des revendications 2 - 4,
dans lequel le ou les états de fonctionnement comprennent le fait que le dispositif de réception d'énergie reçoit une énergie inférieure au seuil ; et
dans lequel, en réponse à la détermination du fait que le dispositif de réception d'énergie reçoit une énergie inférieure au seuil, le circuit de traitement est configuré pour commander l'ensemble de voyants lumineux pour qu'ils émettent en sortie le motif tournoyant.

6. Dispositif de transmission d'énergie selon l'une quelconque des revendications 2 - 5, dans lequel le seuil est un premier seuil, et dans lequel le circuit de traitement est en outre configuré pour :
en réponse à la détermination du fait que le dispositif de réception d'énergie reçoit une énergie sans fil supérieure au premier seuil et reçoit une énergie sans fil inférieure à un deuxième seuil, amener l'ensemble de voyants lumineux à émettre une impulsion avec un premier modèle d'impulsion, et
en réponse à la détermination du fait que le dispositif de réception d'énergie reçoit une énergie sans fil supérieure au deuxième seuil, et reçoit une énergie sans fil inférieure à un troisième seuil, amener l'ensemble de voyants lumineux à émettre une impulsion avec un second modèle d'impulsion.

7. Dispositif de transmission d'énergie selon l'une quelconque des revendications 2 - 6, dans lequel le circuit de traitement est en outre configuré pour, en réponse à la détermination du fait que le dispositif de réception d'énergie reçoit une énergie sans fil supérieure au premier seuil et reçoit une énergie sans fil inférieure à un deuxième seuil, amener le circuit audio à émettre une alerte sonore avec un premier motif sonore.

8. Dispositif de transmission d'énergie selon l'une quelconque revendication 4, dans lequel l'ensemble de voyants lumineux configurés pour éclairer la commande est un premier ensemble de voyants lumineux, le dispositif de transmission d'énergie comprenant en outre un second ensemble de voyants lumineux séparé de la touche, dans lequel le second ensemble de voyants lumineux fournit :
une indication d'un niveau de charge pour un premier dispositif de stockage électrique du dispositif de transmission de puissance à un premier moment ; et
une indication d'un niveau de charge pour un second dispositif de stockage électrique du dispositif de réception d'énergie à un second moment.

9. Dispositif de transmission d'énergie selon l'une quelconque revendication 8, dans lequel le circuit de traitement est en outre configuré pour amener un voyant lumineux du second ensemble de voyants lumineux à émettre en sortie une indication que le premier dispositif de stockage électrique du dispositif de transmission d'énergie ne dispose pas suffisamment d'énergie électrique disponible pour fournir une énergie sans fil au dispositif de réception d'énergie.

10. Dispositif de transmission d'énergie selon l'une quelconque des revendications 2 - 9, dans lequel, en réponse à la détermination du fait que le dispositif de réception d'énergie reçoit une énergie sans fil supérieure au seuil, le circuit de traitement est configuré pour commander l'ensemble de voyants lumineux pour qu'ils émettent une impulsion.

11. Système comprenant :
le dispositif de transmission d'énergie selon l'une quelconque des revendications 1 à 10, et
le dispositif de réception d'énergie.

12. Système selon la revendication 11, comprenant en outre un dispositif informatique externe configuré pour communiquer sans fil avec le dispositif de transmission d'énergie et le dispositif de réception d'énergie, le dispositif informatique externe comprenant une interface utilisateur à écran tactile.

13. Système selon les revendications 11 et 12, comprenant en outre un dispositif de transfert d'énergie filaire configuré pour transférer l'énergie électrique au dispositif de transmission d'énergie.

14. Système selon la revendication 13, dans lequel l'ensemble de voyants lumineux est un premier ensemble de voyants lumineux, le dispositif de transmission d'énergie comprenant en outre un second ensemble de voyants lumineux, dans lequel le second ensemble de voyants lumineux fournit :
une indication d'un niveau de charge pour une première unité de stockage électrique du dispositif de transmission d'énergie à un premier moment ; et
une indication d'un niveau de charge pour une seconde unité de stockage électrique du dispositif de réception d'énergie à un second moment.

15. Système selon la revendication 14, dans lequel le second ensemble de voyants lumineux fournit :
l'indication du niveau de charge pour la première unité de stockage électrique du dispositif de transmission d'énergie lorsque le dispositif de transmission d'énergie est connecté électriquement au dispositif de transfert d'énergie filaire ; et
l'indication du niveau de charge pour la seconde unité de stockage électrique du dispositif de réception d'énergie lorsque le dispositif de transmission d'énergie est déconnecté du dispositif de transfert d'énergie filaire.
